(19) Europäisches Patentamt
European Patent Office
Office européen des brevets

(11) Publication number: **0 146 374 B1**

(12) **EUROPEAN PATENT SPECIFICATION**

(45) Date of publication of patent specification: **13.06.90**

(51) Int. Cl.[5]: **C 07 C 29/03**, C 07 C 31/20, C 07 C 35/20, C 07 C 35/14, C 07 C 35/06, C 07 C 33/26

(21) Application number: **84308775.0**

(22) Date of filing: **14.12.84**

(54) **Process for the preparation of vicinal diols soluble in water.**

(30) Priority: **16.12.83 IT 2420383**

(43) Date of publication of application:
**26.06.85 Bulletin 85/26**

(45) Publication of the grant of the patent:
**13.06.90 Bulletin 90/24**

(84) Designated Contracting States:
**BE CH DE FR GB LI NL**

(56) References cited:
**EP-A-0 109 273**
**GB-A-2 055 821**
**US-A-3 156 709**

(73) Proprietor: **Montedison S.p.A.**
**31, Foro Buonaparte**
**I-20121 Milan (IT)**

(72) Inventor: **Venturello, Carlo**
**135, Via XXIII Marzo**
**I-Novara (IT)**
Inventor: **Gambaro, Mario**
**5, Via Borrini**
**I-28067 Pernate Novara (IT)**

(74) Representative: **Whalley, Kevin et al**
**MARKS & CLERK 57/60 Lincoln's Inn Fields**
**London WC2A 3LS (GB)**

**EP 0 146 374 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of vicinal diols soluble in water. More particularly, this invention relates to a process for the preparation of such diols by direct catalytic hydroxylation of the corresponding olefines with hydrogen peroxide.

The vicinal hydrosoluble diols are products of particular interest for the chemical industry. They are mainly used as intermediates in the pharmaceutical industry, in photography, in the textile industry, in the cosmetic industry, for herbicides, in polymers and in additives for plastics materials. For instance, 1-phenyl-1,2-ethandiol is used for the production of 2-phenylethanol (essence of roses), and the diester of trans-1,2-cyclohexandiol with lauric acid is used as a plasticizer for polyvinyl chloride.

Various different processes are known for the direct hydroxylation of the olefines with $H_2O_2$. Some of these processes are based on the use of organic peracids such as peracetic acid or performic acid, in general prepared *in situ,* starting from $H_2O_2$ and the corresponding acids. Other processes are based on the use in catalytic quantities of metal oxides such as $OsO_4$ or $H_2WO_4$.

None of these processes is, however, free of drawbacks. Thus, in the case of the hydroxylation of olefines catalyzed by $OsO_4$, there occur serious problems as far as the cost and the toxicity of the catalyst is concerned and because of the necessity of operating with anhydrous $H_2O_2$ solutions; moreover, the yields are not always satisfactory.

The hydroxylation of olefines catalized by tungstic acid is found to be fully satisfactory on the practical level, only when starting from hydrosoluble olefinic compounds such as allyl alcohol, maleic acid and fumaric acid. With almost all other substrates, because of the necessity of operating in the presence of suitable solvents (such as acetic acid) capable of solubilizing both reactants, there arises the economically rather burdensome problem of the isolation and purification of the product from the reaction mixture. The problem is complicated, in the case of the use of acetic acid as a solvent, by the necessity of saponifying with NaOH the intermediate hydroxy acetate that has formed in the reaction medium. Still another limitation of the method consists in the moderate effectiveness of the catalyst in the monophasic aqueous-organic system when using not particularly active substrates.

The problem of the isolation of the diol, with all the operations involved in it, constitute also the critical point of the process via organic peracids, which is the most widely used on an industrial scale.

In fact it is not possible to obtain directly by this way the desired product with a sufficient degree of purity; there are required burdensome preliminary treatments of the reaction mixture, which treatments comprise:

1) removing by distillation (together with the unreacted olefine) and/or neutralization with sodium hydroxide considerable quantities of organic acid which must somehow be recovered;

2) successive extraction operations with a solvent of the diol from the resulting reaction residue and/or fractional distillation operations.

GB—A—2055821 discloses a process for oxidising olefins by mixing an aqueous phase containing $H_2O_2$ with an organic phase containing the olefin in the presence of a 2-component catalyst system; this process results in the production of epoxides.

Thus, one aim of the present invention is that of providing an economically convenient process for the preparation of vicinal hydrosoluble diols by direct hydroxylation of the corresponding olefines with $H_2O_2$, which will allow the diols to be obtained directly with a high degree of purity, thus avoiding the necessity of laborious treatments of the reaction mixture.

Another aim of the invention is that of providing a catalytic process that will, being catalytic, allow the use of organic reactants, such as acetic acid or formic acid, used in considerable quantities, to be avoided.

Still another aim of the invention is that of providing a process that uses a catalyst of low cost.

A yet further aim of the invention is that of providing a process that will allow the use of dilute aqueous solutions of $H_2O_2$, thereby obtaining advantages both from the point of view of economy as well as with regard to operational safety, with respect to the previously known processes wherein usually there is used very concentrated $H_2O_2$.

The present invention provides a process for the preparation of a hydrosoluble vicinal diol by means of catalytic hydroxylation of the corresponding olefine with $H_2O_2$, wherein an olefine, optionally substituted with one or more functional groups inert under the reaction conditions and whose corresponding vicinal diol is soluble in water, is reacted, under vigorous stirring, with $H_2O_2$, at a temperature from 0°C to 120°C, and at a pressure from 1 to 100 atmospheres, in a biphasic aqueous liquid/organic liquid system consisting of an acid aqueous phase having a pH from 0 to 1.5 and containing $H_2O_2$ and an organic phase containing:

1) the said olefine;

2) optionally a solvent immiscible with the aqueous phase; and

3) a catalyst substantially consisting of a peroxy-phospho-tungstate and/or a peroxy-arseno-tungstate of the formula: $Q_3XW_4O_{24-2n}$ wherein:

Q represents an onium $(RR_1R_2R_3M)^+$ cation in which M is selected from N, P, As and Sb, and R, $R_1$, $R_2$ and $R_3$, which may be the same as or different from each other, represent hydrogen atoms or hydrocarbon groups, the radicals R, $R_1$, $R_2$ and $R_3$ having in total from 20 to 70 carbon atoms;

X is P or As;

and n is 0, 1 or 2.

The hydroxylation reaction of the olefines according to the present invention may be represented by the following equation:

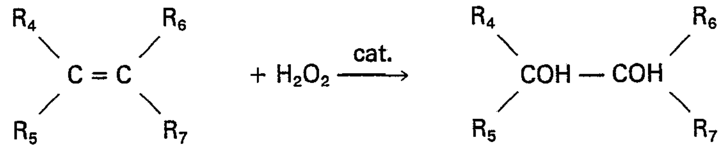

(1)

wherein: $R_4$, $R_5$, $R_6$ and $R_7$, which may be the same as or different from each other, are hydrogen atoms or hydrocarbon groups (such as alkyls, aryls or alkylaryls), optionally carrying one or more functional groups inert under reaction conditions.

The hydrocarbon groups and the optional functional groups are such as to ensure the solubility in water of the corresponding diols.

Moreover, a hydrocarbon radical $R_4$ or $R_5$ attached to an ethylenic carbon, joining a hydrocarbon radical $R_6$ or $R_7$ attached to the other ethylenic carbon, may form an alkenylic cycle containing from 4 to 7 carbon atoms.

The functional groups inert under reaction conditions, which may be the same as or different from each other, are for instance Cl, F, OH, $OCH_3$ and COOH. In general there are from 0 to 3 such groups.

Catalysts $Q_3XW_4O_{24-2n}$ and their method of preparation are described in European Patent Application No. 109,273. Such catalysts are used to oxidise olefins with $H_2O_2$.

These catalysts may be prepared as follows:

First there are reacted tungstic acid or an alkali metal tungstate, or phosphoric acid or an alkali metal phosphate, or a corresponding arsenic compound, and $H_2O_2$, contained in an acid aqueous phase. The reaction product thus obtained is then reacted with an onium salt contained in an organic solvent immiscible with water. The onium salt $Q^+Y^-$ consists of the already defined $Q^+$ cation and an inorganic $Y^-$ anion stable under the reaction conditions, such as for example $Cl^-$, $HSO_4^-$ or $NO_3^-$. The acid aqueous phase has a pH below 1.5; if necessary, the pH is adjusted with a mineral acid (for instance $H_4SO_4$ or HCl).

The reaction between the above indicated inorganic reactants in general is carried out at a temperature from 20°C to 80°C; thereupon there is added, preferably at room temperature, the onium salt in its solvent (for instance dichloroethane or benzene); the stirring of the biphasic mixture is carried on for 15—20 minutes.

The molar ratios between the reactants are usually: for each gram atom of X (P or As), there are used 4 grams atoms of W and up to 2 mols of onium salt; as far as the $H_2O_2$ is concerned, it is sufficient to use from 2.5 to 6 mols of $H_2O_2$ for each gram atom of W.

If the product that is formed is in the solid state, it will be directly separated from the biphasic mixture, for instance by filtering. If the product is in the liquid state, the organic phase will be separated, filtered and evaporated under vacuum at from 40°C to 50°C, thereby obtaining the catalyst in the form of either a solid or a thick oil.

In the onium $(RR_1R_2R_3M)^+$ cation, M is selected from N, P, As and Sb. Preferably there are used catalysts in which M is either N or P.

Radicals R, $R_1$, $R_2$ and $R_3$ have in total from 20 to 70 carbon atoms. Preferably there are used catalysts in which said total is from 25 to 40 carbon atoms.

There may also be used mixtures of $Q_3XW_4O_{24-2n}$ catalysts. The mixtures of such a type may be obtained, for instance, starting from commercial mixtures of onium salts, for example from the one known by the commercial name of ARQUAD 2HT (dimethyl[dioctadecyl (75%) + dihexadecyl (25%)] ammonium chloride).

The hydroxylation reaction is conducted according to the double-phase technique. The organic phase contains the olefine, the catalyst and, possibly, a solvent immiscible with the aqueous phase. When no solvent is used, there will be used a suitable excess of olefine. The use or not of a solvent depends on the nature of the catalyst and of the olefine. In fact, the catalyst may sometimes be insoluble in the olefine. On the other hand, if the olefine is highly reactive, it may be convenient to use a solvent.

As solvents for the organic phase, when present, there are used inert solvents immiscible with the aqueous phase. There may be used, for instance: 1) aromatic hydrocarbons such as benzene, toluene and xylenes; 2) chlorinated hydrocarbons, such as dichloromethane, trichloromethane, chloroethane, chloropropanes, dichloroethanes, trichloroethanes, tetrachloroethanes, dichloropropanes, trichloropropanes, tetrachloropropanes, and chlorobenzene; and 3) alkyl esters, such as ethyl acetate. There may also be used suitable mixtures of these solvents.

Olefinic compounds that may be conveniently used as starting substances are, for example: styrene, the various vinyltoluenes (ortho-, metha-, para-), alpha-methylstyrene, cyclopentene, cyclohexene, cycleheptene, tetramethylethylene, 1-hexene, 1-pentene, 2-butene, propylene, allyl chloride, cinnamyl alcohol, isoeugenol, isosafrole and beta-methylstyrene.

When starting from cyclo-olefines, the cycloalkandiols have a trans configuration.

The pH of the aqueous phase is from 0 to 1.5. The aqueous phase may be acidified with mineral or organic acids, for example sulphuric acid, phosphoric acid and sulphonic acids; preferably there is used sulphuric acid.

The $H_2O_2$ concentration in the aqueous phase is preferably from 1% to 10% by weight, more preferably from about 2% to about 4% by weight.

The operational temperature is determined by the reactivity and by the nature of the olefine as well as by the stability of the hydrogen peroxide and of the catalyst used. In general the reaction is preferably conducted at a temperature from 20°C to 120°C, more preferably from about 40°C to about 90°C.

The operational pressure is usually atmospheric pressure. However, in the case of low-boiling olefins it will be necessary to operate at a pressure sufficient (up to $10^7$ Pa, 100 atm.) to maintain the olefine in the liquid state.

The reactants, that is the olefine and the $H_2O_2$, may be used according to molar ratios corresponding substantially to the stoichiometry of the reaction. However, whether in the presence or in the absence of a suitable solvent, it will be advantageous and preferable to use an excess of olefine, in general corresponding to a molar ratio of olefine/$H_2O_2$ of from 1.5:1 to 5:1.

The catalyst is preferably used in an amount of from 0.005 to 0.1 gram atom of W per mol of $H_2O_2$, more preferably from about 0.01 to about 0.03 gram atom of W per mol of $H_2O_2$.

Whenever a solvent is used in the organic phase, the concentration of the olefine in the organic phase will generally be from 5% to 95% by weight, more preferably from about 40% to about 80% by weight.

The duration of the reaction depends on the nature and on the quantity of catalyst used and on the type of olefine used. In general, a time of from 1 to 5 hours will be sufficient for completing the reaction.

At the end of the reaction, the vicinal diols that have formed may be directly recovered from the aqueous phase by using conventional techniques, after the preliminary destruction, according to known methods, of the still present residual $H_2O_2$.

For example the following procedure may be used: the residual $H_2O_2$ is destroyed with bisulphite; the aqueous phase is then neutralised and evaporated to dryness under a reduced pressure; the diol is then extracted from the dry residue by means of a suitable solvent, for instance ether, acetone or ethyl acetate. In certain cases the diol will be partially present in the organic phase, and it will then be convenient to repeatedly extract that phase with water in order to complete the recovery of the diol.

The invention will be further described with reference to the following illustrative Examples.

Example 1

Into a 300 ml reactor, fitted with a reflux coolant, a thermometer and a mechanical stirrer, there were introduced 30 ml of styrene (261 mmols), 1.70 g of catalyst $\{[C_{18}H_{37}(75\%) + C_{16}H_{33}(25\%)]_2N(CH_3)_2\}_3 PW_4O_{22}$ (equal to 2,5 mmols of W), and an aqueous solution of $H_2O_2$ obtained by dissolving 8.5 ml of $H_2O_2$ in a 40% concentration by weight/volume (400 g/lt.) (100 mmols) in 160 ml of $H_2O$ and by bringing the pH value of the resulting solution to 1.5 with a 30% by weight $H_2SO_4$.

The biphasic mixture resulting therefrom was quickly brought, under vigorous stirring, to 60°C and then maintained at this temperature for 75 minutes. After cooling down, there were added 4 ml of a 30% by weight $H_2SO_4$ in order to facilitate the separation of the phases. Thereupon there was separated the aqueous phase which was then filtered on a paper filter and then additioned with sodium metabisulphite in order to destroy the $H_2O_2$ still present.

The solution was thereupon brought up to a pH of about 8 by the addition of solid $Na_2CO_3$, whereafter it was evaporated to dryness.

The solid residue was then extracted with ethyl ether (100 ml) and kept stirred under reflux. This treatment was repeated three times.

Then, by evaporation of the ether solution, there were obtained 11.70 g of 1-phenyl-1,2-ethandiol as a white solid (gas chromatographic titre: 99%). The yield of diol (expressed as 100% diol) was equal to 84% (calculated on the loaded $H_2O_2$).

By extraction of the organic phase with water acidified with $H_2SO_4$ there could be obtained further 0.20—0.25 g of sufficiently pure diol.

Example 2

Example 1 was repeated, except that there was used alpha-methylstyrene (34 ml; 260 mmols) instead of styrene.

Thereby there were obtained 12.60 g of 2-phenyl-1,2-propandiol as a white solid (gas chromatographic titre 99%). The yield of diol (expressed as 100% diol) was equal to 82% (calculated on the loaded $H_2O_2$).

By treating the organic phase there could be obtained further 0.4—0.5 g of sufficiently pure diol.

Example 3

Into a 250 ml reactor, fitted with a reflux coolant, a thermometer and a mechanical stirrer, there were introduced 15.35 ml of cyclohexene (150 mmols), 10 ml of benzene, 0.85 g of the same catalyst as used in example 1 (equal to 1.25 mmols of W), and an aqueous solution of $H_2O_2$ obtained by dissolving 8.5 ml of a 40% weight/volume $H_2O_2$ (100 mmols) in 80 ml of $H_2O$ and by then bringing the pH value of the resulting solution to 1.5 with a 30% by weight $H_2SO_4$.

The biphasic mixture resulting therefrom was rapidly brought up to 70°C, under vigorous stirring, and was then maintained at this temperature for 60 minutes.

There was then followed the procedure as described in example 1, except that the solid residue was extracted with acetone (3 × 150 ml) at 50°C instead of with ether.

Thereby there were obtained 10.85 g of trans-1,2-cyclohexandiol as a white solid (gas chromatographic titre: 99%). The yield of diol (expressed as 100% diol) was equal to 92% (calculated on the loaded $H_2O_2$).

Example 4

Example 3 was repeated, but using instead the catalyst: $[(C_8H_{17})_3NCH_3]_3PW_4O_{22}$ (0.695 g, equal to 1.25 mmols of W).

There were obtained 10.77 g of trans-1,2-cyclohexandiol as a white solid (gas chromatographic titre: 99%). The yield of diol (expressed as 100% diol) was equal to 92% (calculated on the loaded $H_2O_2$).

Example 5

Example 3 was repeated, but using cyclopentene (13.2 ml; 150 mmols) instead of cyclohexene, 1.365 g of the catalyst of example 1 (equal to 2 mmols of W), and operating at 55°C (bath temperature) for 2 hours.

Thereby there were obtained 9.40 g of trans-1,2-cyclopentandiol in the form of a thick oil that solidified slowly (gas chromatographic titre: 98%). The yield of diol (expressed as 100% diol) was equal to 90.3% (calculated on the loaded $H_2O_2$).

Example 6

Example 5 was repeated, but using cycloheptene (17.5 ml; 150 mmols) instead of cyclohexene, and operating at 60°C for 2 hours.

Thereby there were obtained 10.48 g of trans-1,2-cycloheptandiol as a white solid (gas chromatographic titre: 96%). The yield of diol (expressed as 100% diol) was equal to 77.4% (calculated on the loaded $H_2O_2$).

Through the treatment of the organic phase there could be obtained further about 0.7 g of diol (gas chromatographic titre: 98.5%), which corresponded to a 5% yield.

Example 7

Into a 300 ml reactor, fitted with a reflux coolant, a thermometer and a mechanical stirrer, there were introduced 25 ml of 1-hexene (200 mmols), 1.67 g of the same catalyst as used in example 4 (equal to 3 mmols of W), 20 ml of 1,2-dichloroethane, and an aqueous solution of $H_2O_2$ obtained by dissolving 8.5 ml of a 40% weight/volume $H_2O_2$ (100 mmols) in 150 ml of $H_2O$ and then bringing the pH value of the resulting solution to 1 with a 30% by weight $H_2SO_4$.

The biphasic mixture thus resulting was then brought to reflux under vigorous stirring (temperature of the bath: 65°C) and was then kept at this temperature for 3 (three) hours. At the end there was added just a little ether (10—15 ml) in order to facilitate the separation of the phases.

The organic phase was thereupon extracted with $H_2O$ (4 × 70 ml). The aqueous extract was then joined with the aqueous phase. The resulting aqueous solution, after destruction of the residual $H_2O_2$, was brought up to a pH of about 8 with solid $Na_2CO_3$ and then evaporated to dryness. The residue was then extracted with acetone (3 × 150 ml).

By evaporation of the acetone solution there were obtained 7.92 g of 1,2-hexandiol in the form of an oil (gas chromatographic titre: 96%). The yield of diol (expressed as 100% diol) was equal to 64% (calculated on the loaded $H_2O_2$).

Example 8

Example 7 was repeated, but using allyl chloride (20.5 ml; 250 mmols) instead of 1-hexene, and operating at 66°—68° (temperature of the bath) for 3½ hours. At the end of the operation, the aqueous phase was treated as in example 7. The product obtained by evaporation of the acetone solution was eluted on a silica column with ether.

By evaporation of the solvent there were obtained 7.87 g of 3-chloro-1,2-propandiol in the form of an oil (gas chromatographic titre: 98.7%). The yield of diol (expressed as 100% diol) was equal to 70% (calculated on the loaded $H_2O_2$).

Example 9

Into a 1 litre autoclave, with a glass lining and fitted with a magnetic stirrer, there were introduced 3.34 g of the same catalyst as used in example 4 (equal to 6 mmols of W), 40 ml of 1,2-dichloroethane, and an aqueous solution of $H_2O_2$ obtained by dissolving 17 ml of a 40% weight/volume $H_2O_2$ (200 mmols) in 160 ml of $H_2O$ and then bringing the pH of the resulting solution to 1 with a 30% by weight $H_2SO_4$.

After removing the air from the autoclave by applying a vacuum, there were loaded in the autoclave 42 g of propylene. The mixture was then heated up to 70°C, during about 1 hour, under vigorous stirring, and thereby there was attained a pressure of 19 × $10^5$ Pa (19 atmospheres), and the reaction mixture was maintained at that temperature for one hour.

At the end of this operation, after cooling down (in about 1 hour) and after the removal of the gases, the autoclave was discharged. The aqueous phase was treated as in example 7.

There were obtained 8.80 g of 1,2-propandiol in the form of an oil (gas chromatographic titre: 97.4%). The yield in diol (expressed as 100% diol) was equal to 56% (calculated on the loaded $H_2O_2$).

**Claims**

1. A process for the preparation of a hydrosoluble vicinal diol by means of catalytic hydroxylation of the corresponding olefine with $H_2O_2$, wherein an olefine, optionally substituted with one or more functional groups inert under the reaction conditions and whose corresponding vicinal diol is soluble in water, is reacted, under vigorous stirring, with $H_2O_2$, at a temperature from 0°C to 120°C, and at a pressure from 1 to 100 atmospheres, in a biphasic aqueous liquid/organic liquid system consisting of an acid aqueous phase having a pH from 0 to 1.5 and containing $H_2O_2$ and an organic phase containing:

1) the said olefine;

2) optionally a solvent immiscible with the aqueous phase; and

3) a catalyst substantially consisting of a peroxy-phospho-tungstate and/or a peroxy-arseno-tungstate of the formula: $Q_3XW_4O_{24-2n}$ wherein:

Q represents an onium $(RR_1R_2R_3M)^+$ cation in which M is selected from N, P, As and Sb, and R, $R_1$, $R_2$ and $R_3$, which may be the same as or different from each other, represent hydrogen atoms or hydrocarbon groups, the radicals R, $R_1$, $R_2$ and $R_3$ having in total from 20 to 70 carbon atoms;

X is P or As;

and n is 0, 1 or 2.

2. A process as claimed in claim 1, characterized in that, in the onium $(RR_1R_2R_3M)^+$ cation M is N or P.

3. A process as claimed in claim 1 or 2, characterized in that, in the onium $(RR_1R_2R_3M)^+$ cation, the radicals R, $R_1$, $R_2$ and $R_3$ have in total from 25 to 4 carbon atoms.

4. A process as claimed in any of claims 1 to 3, characterized in that the solvent immiscible with the aqueous phase, when present, is selected from aromatic hydrocarbons, chlorinated hydrocarbons and alkyl esters.

5. A process as claimed in any of claims 1 to 4, characterized in that the concentration of $H_2O_2$ in the aqueous phase is from 1% to 10% by weight.

6. A process as claimed in any of claims 1 to 5, characterized in that the reaction temperature is from 20°C to 120°C.

7. A process as claimed in claim 6, characterized in that the reaction temperature is from 40°C to 90°C.

8. A process as claimed in any of claims 1 to 7, characterized in that the molar ratio olefine/$H_2O_2$ is from 1.5:1 to 5:1.

9. A process as claimed in any of claims 1 to 8, characterized in that the catalyst is used in amount of from 0.005 to 0.1 gram atom of W per mol of $H_2O_2$.

**Patentansprüche**

1. Verfahren zur Herstellung eines wasserlöslichen vicinalen Diols mittels katalytischer Hydroxylierung des entsprechenden Olefins mit $H_2O_2$, bei welchem ein Olefin, das gegebenenfalls mit einer oder mehreren unter den Reaktionsbedingungen inerten Gruppen substituiert ist und dessen entsprechendes vicinales Diol in Wasser löslich ist, unter kräftigem Rühren bei einer Temperatur von 0°C bis 120°C und einem Druck von 1 bis 100 Atmosphären in einem zweiphasigen System wäßrige Flüssigkeit/organische Flüssigkeit mit $H_2O_2$ umgesetzt wird, wobei das zweiphasige System besteht aus einer sauren wäßrigen Phase mit einem pH von 0 bis 1,5, die $H_2O_2$ enthält, und einer organischen Phase, die enthalt:

1) das besagte Olefin;

2) gegebenenfalls ein mit der wäßrigen Phase nicht mischbares Lösungsmittel; und

3) einen Katalysator, der im wesentlichen besteht aus einem Peroxy-Phospho-Wolframat und/oder einem Peroxy-Arseno-Wolframat der Formel: $Q_3XW_4O_{24-2n}$ in welcher:

Q ein Oniumkation $(RR_1R_2R_3M)^+$ darstellt, wobei M ausgewählt ist aus N, P As und Sb, und R, $R_1$, $R_2$ und $R_3$, die gleich oder verschieden voneinander sein können, Wasserstoffatome oder Kohlenwasserstoffgruppen darstellen und die Gruppen R, $R_1$, $R_2$ und $R_3$ insgesamt 20 bis 70 Kohlenstoffatome aufweisen;

X für P oder As steht; und

n 0, 1 oder 2 ist.

2. Verfahren nach Anspruch 1, dadurch gekennzeichnet, daß in dem Oniumkation $(RR_1R_2R_3M)^+$ M für N oder P steht.

3. Verfahren nach Anspruch 1 oder 2, dadurch gekennzeichnet, daß in dem Oniumkation $(RR_1R_2R_3M)^+$ die Gruppen R, $R_1$, $R_2$ und $R_3$ insgesamt 25 bis 40 Kohlenstoffatome aufweisen.

4. Verfahren nach irgendeinem der Ansprüche 1 bis 3, dadurch gekennzeichnet, daß das mit der wäßrigen Phase nicht mischbare Lösungsmittel, wenn es anwesend ist, ausgewählt wird aus aromatischen Kohlenwasserstoffen, chlorierten Kohlenwasserstoffen und Alkylestern.

5. Verfahren nach irgendeinem der Ansprüche 1 bis 4, dadurch gekennzeichnet, daß die Konzentration von $H_2O_2$ in der wäßrigen Phase von 1 bis 10 Gewichtsprozent beträgt.

6. Verfahren nach irgendeinem der Ansprüche 1 bis 5, dadurch gekennzeichnet, daß die Reaktionstemperatur 20 bis 120°C beträgt.

7. Verfahren nach Anspruch 6, dadurch gekennzeichnet, daß die Reaktionstemperatur 40 bis 90°C beträgt.

8. Verfahren nach irgendeinem der Ansprüche 1 bis 7, dadurch gekennzeichnet, daß das Molverhältnis Olefin/$H_2O_2$ 1,5:1 bis 5:1 beträgt.

9. Verfahren nach irgendeinem der Ansprüche 1 bis 8, dadurch gekennzeichnet, daß der Katalysator in einer Menge von 0,005 bis 0,1 Grammatom W pro Mol $H_2O_2$ eingesetzt wird.

**Revendications**

1. Un procédé pour la préparation d'un diol vicinal hydrosoluble par hydroxylation catalytique de l'oléfine correspondante avec du $H_2O_2$, caractérisé en ce qu'une oléfine, éventuellement substituée avec un ou plusiers groupes fonctionnels inertes dans les conditions de la réaction et dont le diol vicinal correspondant est soluble dans l'eau, est traitée sous vigoureuse agitation avec du $H_2O_2$, à une température de 0° à 120°C, sous une pression de 1 à 100 atmosphères, dans un système de liquide aqueux/liquide organique à deux phases consistant en une phase aqueuse acide ayant un pH de 0 à 1,5 et contenant du $H_2O_2$ et en une phase organique contenant:

1) ladite oléfine;

2) éventuellement un solvant non-miscible à la phase aqueuse; et

3) un catalyseur consistant pratiquement en un peroxy-phospho-tungstate et/ou un peroxy-arsénio-tungstate de formule $Q_3XW_4O_{24-2n}$ dans laquelle:

Q est un cation onium $(RR_1R_2R_3M)^+$ dans lequel M est choisi entre N, P, As et Sb; et R, $R_1$, $R_2$ et $R_3$ qui peuvent être identiques ou différents les uns des autres, sont des atomes d'hydrogène ou des groupes hydrocarbonés, les radicaux R, $R_1$, $R_2$ et $R_3$ ayant au total de 20 à 70 atomes de carbone;

X est P ou As;

et n est 0, 1 ou 2.

2. Un procédé suivant la revendication 1, caractérisé en ce que dans le cation onium $(RR_1R_2R_3M)^+$, M est N ou P.

3. Un procédé suivant la revendication 1 ou 2, caractérisé en ce que dans le cation onium $(RR_1R_2R_3M)^+$, les radicaux R, $R_1$, $R_2$ et $R_3$ ont au total de 25 à 40 atomes de carbone.

4. Un procédé suivant l'une quelconque des revendications 1 à 3, caractérisé en ce que le solvant non-miscible à la phase aqueuse, lorsqu'il est présent, est choisi parmi des hydrocarbures aromatiques, des hydrocarbures chlorés et des esters alkyliques.

5. Un procédé suivant l'une quelconque des revendications 1 à 4, caractérisé en ce que la concentration de $H_2O_2$ dans la phase aqueuse est de 1% à 10% en poids.

6. Un procédé suivant l'une quelconque des revendications 1 à 5, caractérisé en ce que la température de réaction est de 20° à 120°C.

7. Un procédé suivant la revendication 6, caractérisé en ce que la température de réaction est de 40° à 90°C.

8. Un procédé suivant l'une quelconque des revendications 1 à 7, caractérisé en ce que le rapport molaire oléfine/$H_2O_2$ est de 1,5/1 à 5/1.

9. Un procédé suivant l'une quelconque des revendications 1 à 8, caractérisé en ce que le catalyseur est utilisé en une quantité de 0,005 à 0,1 atome gramme de W par mole de $H_2O_2$.